(19) Europäisches Patentamt
European Patent Office
Office européen des brevets

(11) **EP 3 417 823 B1**

(12) **EUROPEAN PATENT SPECIFICATION**

(45) Date of publication and mention
of the grant of the patent:
**17.02.2021 Bulletin 2021/07**

(51) Int Cl.:
***A61B 18/18*** (2006.01)

(21) Application number: **18178570.0**

(22) Date of filing: **19.06.2018**

(54) **TAPERED TUBULAR MEMBERS FOR LAPAROSCOPIC MICROWAVE ABLATION INSTRUMENTS**

KONISCHE ROHRFÖRMIGE ELEMENTE FÜR LAPAROSKOPISCHE MIKROWELLENABLATIONSINSTRUMENTE

ÉLÉMENTS TUBULAIRES CONIQUES POUR INSTRUMENTS D'ABLATION LAPAROSCOPIQUE PAR MICRO-ONDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.06.2017 US 201715628376**

(43) Date of publication of application:
**26.12.2018 Bulletin 2018/52**

(73) Proprietor: **Covidien LP**
**Mansfield, MA 02048 (US)**

(72) Inventors:
• **BRANNAN, Joseph D.**
**Lyons, CO 80540 (US)**
• **LARSON, Eric W.**
**Littleton, CO 80127 (US)**

(74) Representative: **Maschio, Antonio**
**Maschio & Soames IP Limited**
**30 Carlton Crescent**
**Southampton SO15 2EW (GB)**

(56) References cited:
GB-A- 2 545 179          US-A- 5 967 984
US-A1- 2004 169 572     US-A1- 2014 290 830

**Description**

BACKGROUND

Conventional microwave ablation systems are used during laparoscopic surgical procedures to treat target tissue, such as a tumor located within the abdomen or pelvis, by delivering microwave energy to the target tissue by way of a percutaneous microwave ablation instrument. The instrument typically includes an elongated tubular member, which has a needle at a distal tip, and which serves as a conduit within which the microwave energy from an energy source, such as a generator, is guided through an incision in the patient and toward the target tissue. The length of the tubular member varies based on application. In some cases, microwave instruments with relatively long tubular members are employed to reach target tissue that is located deep within the body of the patient. Unfortunately, as the length of the tubular member increases, the flexure of the tubular member during operation also increases, thereby reducing or distorting the tactile feedback from a distal tip of the instrument to a handle located at a proximal portion of the instrument. Although stiffness of the tubular member may be increased by increasing a diameter of the tubular member along its entire longitudinal axis, doing so would also result in larger needle track sizes, an increased risk of bleeding and damage to the target tissue, and an increased post-surgery healing time. In this context, reference is made to the following documents: GB2545179 A and US2014/0290830 A1.

SUMMARY

The invention is defined in the appended set of claims. In one aspect of this disclosure, a tubular member for a laparoscopic microwave ablation instrument is described. The tubular member includes a proximal portion, a distal portion, and an intermediate portion, which is interposed between the proximal portion and the distal portion. A maximum outer diameter of the proximal portion is greater than or equal to a maximum outer diameter of the intermediate portion. The maximum outer diameter of the intermediate portion is greater than or equal to a maximum outer diameter of the distal portion. And the maximum outer diameter of the proximal portion is greater than the maximum outer diameter of the distal portion

In embodiments, the intermediate portion has an outer diameter at least a portion of which increases in a direction from the distal portion to the proximal portion.

In embodiments, the proximal portion, the distal portion, and the intermediate portion are each tubular, and the proximal portion, the distal portion, and the intermediate portion each have an equivalent inner diameter that remains uniform along a longitudinal axis of the tubular member.

In embodiments, the outer diameter of the distal portion remains uniform along a longitudinal axis of the distal portion.

In embodiments, the outer diameter of the proximal portion remains uniform along a longitudinal axis of the proximal portion.

In embodiments, a length of the distal portion is predetermined based on an expected insertion depth of the distal portion into a target tissue.

In embodiments, a length of the intermediate portion is predetermined based on an expected insertion depth of the intermediate portion into an abdominal cavity of a patient.

In embodiments, the intermediate portion has an outer diameter that increases linearly in a direction from the distal portion toward the proximal portion.

In embodiments, the intermediate portion includes a plurality of subportions, and wherein each of the plurality of subportions has an outer diameter that increases linearly, at a respective angle, in a direction from the distal portion toward the proximal portion.

In embodiments, the intermediate portion has an outer diameter that increases exponentially in a direction from the distal portion toward the proximal portion.

In embodiments, the intermediate portion includes a plurality of subportions, and each of the plurality of subportions has an outer diameter that increases exponentially, at a respective growth rate, in a direction from the distal portion toward the proximal portion.

In embodiments, the intermediate portion includes a first subportion and a second subportion, the first subportion having an outer diameter that increases linearly in a direction from the distal portion toward the proximal portion, and the second subportion having an outer diameter that increases exponentially in the direction from the distal portion toward the proximal portion.

In embodiments, the first subportion and the second subportion are arranged in a direction from the distal portion to the proximal portion.

In embodiments, near a junction between the first subportion and the second subportion, the outer diameter of the second subportion is greater than the outer diameter of the first subportion, thus forming a step at the junction between the first subportion and the second subportion.

In embodiments, the first subportion and the second subportion are arranged in a direction from the proximal portion to the distal portion.

In embodiments, near a junction between the first subportion and the second subportion, the outer diameter of the first subportion is greater than the outer diameter of the second subportion, thus forming a step at the junction between the first subportion and the second subportion.

In embodiments, the intermediate portion has an outer diameter that increases linearly in a direction from the distal portion toward the proximal portion, and, near a junction between the distal portion and the intermediate portion, the outer diameter of the intermediate portion is greater than an outer diameter of the distal portion, thus forming a step at the junction between the distal portion and the intermediate portion.

In embodiments, near a junction between the intermediate portion and the proximal portion, an outer diameter of the proximal portion is greater than the outer diameter of the intermediate portion, thus forming a step at the junction between the intermediate portion and the proximal portion.

In embodiments, the intermediate portion has an outer diameter that increases exponentially in a direction from the distal portion toward the proximal portion, and, near a junction between the distal portion and the intermediate portion, the outer diameter of the intermediate portion is greater than an outer diameter of the distal portion, thus forming a step at the junction between the distal portion and the intermediate portion.

In embodiments, near a junction between the intermediate portion and the proximal portion, an outer diameter of the proximal portion is greater than the outer diameter of the intermediate portion, thus forming a step at the junction between the intermediate portion and the proximal portion.

BRIEF DESCRIPTION OF THE DRAWINGS

Various aspects and features of the present laparoscopic microwave instrument tubular members are described herein below with references to the drawings, wherein:

FIG. 1 is a cross-sectional view of a known microwave ablation instrument;
FIG. 2 shows a side view of an illustrative embodiment of a portion of a laparoscopic microwave ablation instrument, in accordance with the present disclosure;
FIG. 3 shows an illustrative embodiment of how the instrument of FIG. 2 may be used to treat a target lesion;
FIG. 4 shows a perspective view of a tubular member of the laparoscopic microwave ablation instrument of FIG. 2;
FIG. 5 shows a perspective view of the tubular member of FIG. 3 with a hub cap coupled to a proximal portion thereof;
FIG. 6A and FIG. 6B (collectively, FIG. 6) show side cross-sectional views of the tubular member, hub cap, and o-ring shown in FIG. 5;
FIG. 7 is a side view of the tubular member of FIG. 4, showing an illustrative embodiment of depth indicators that may be indicated thereon;
FIG. 8A and FIG. 8B (collectively, FIG. 8) include additional side cross-sectional views of the tubular member and hub cap shown in FIG. 5, showing additional details regarding the proximal and distal portions thereof; and
FIGS. 9A through 9L (collectively, FIG. 9) depict side views of additional illustrative embodiments of laparoscopic microwave ablation instrument tubular members, in accordance with the present disclosure.

DETAILED DESCRIPTION

The present disclosure is directed to tubular members for laparoscopic microwave ablation instruments. In general, as described in further detail below, the various tubular members of the present disclosure have geometries, such as geometries with outer diameters that vary along the longitudinal axes of the tubular members, that exhibit increased stiffness, even for relatively large tubular member lengths, thereby minimizing the flexure of the tubular member during operation and improving the tactile feedback from a distal tip of the instrument to a handle located at a proximal portion of the instrument. At the same time, the tubular members of the present disclosure also minimize needle track size and invasiveness, particularly at the distal portion where the tubular member enters target tissue, thereby minimizing the risk of bleeding and damage to the target tissue and minimizing post-surgery healing time.

Throughout this description, the term "proximal" refers to the portion of the device or component thereof that is closer to the clinician and the term "distal" refers to the portion of the device or component thereof that is farther from the clinician. The phrases "in an embodiment," "in embodiments," "in some embodiments," or "in other embodiments" may each refer to one or more of the same or different embodiments in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

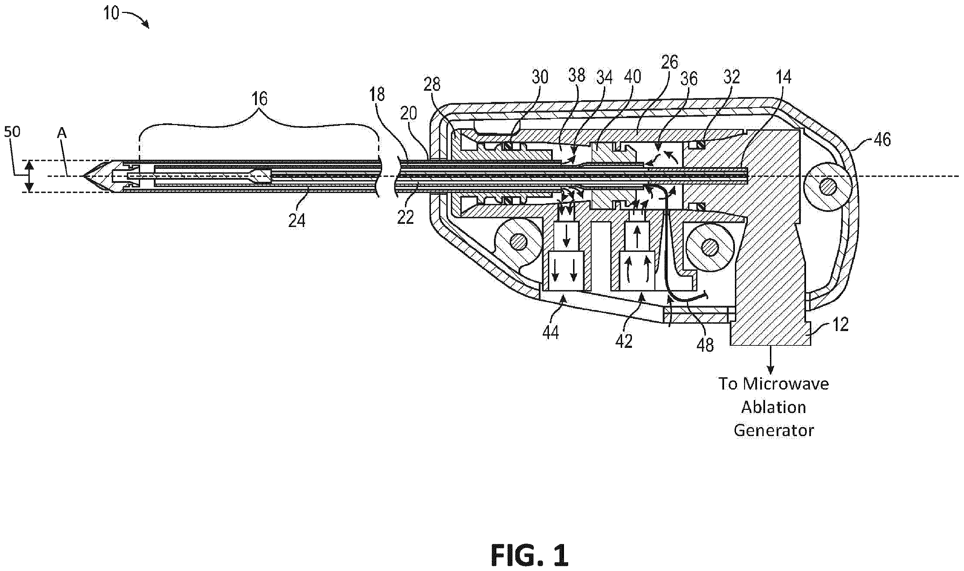

FIG. 1 depicts a known water-jacketed microwave ablation instrument 10 configured for circulating a fluid therethrough. As shown in FIG. 1, the microwave ablation instrument 10 includes a transition 12, which connects via a coaxial cable to a microwave ablation generator (not shown in FIG. 1). The transition 12 allows for a 90° change in direction of the coaxial cable entering the transition 12 to the coaxial cable 14 of the microwave ablation instrument 10. The coaxial cable 14 extends perpendicularly from the transition 12 and concludes at a radiating section 16. The radiating section 16 may take many forms, including monopole, dipole, symmetric and asymmetric configurations. The coaxial cable 14 extends through a first tubular member 18, which is itself housed within a second tubular member 20. Between the coaxial cable 14 and the first tubular member 18 is a first fluid channel 22 and between the first tubular member 18 and the second tubular member 20 is a second fluid channel 24. The transition 12 is received within a first end of a hub 26, a hub cap 28 is received at a second end of the hub 26, and is itself designed to receive and secure the second tubular member 20. O-rings 30 and 32 formed on the hub cap 28 and the transition 12, form seals creating a watertight compartment 34 there between.

Further, as shown in FIG. 1, the watertight compartment 34 is separated into inflow chamber 36 and outflow chamber 38 by hub divider 40. The hub divider 40 receives the first tubular member 18 and maintains it in alignment with the second tubular member 20. The hub divider 40 is formed of an elastomeric material and forms a seal around the first tubular member 18 which, in combination with a compression fit within the hub 26, restricts the egress of fluid in inflow chamber 36 to the first fluid channel 22, and prevents fluid returning through second fluid channel 24 and entering outflow chamber 38 from re-entering the inflow chamber 36. Also shown in FIG. 1 are inflow port 42 and outflow port 44 which connect to inflow chamber 36 and outflow chamber 38, respectively. A wire 48 is depicted extending through the hub 26 and inflow chamber 36 and entering the first tubular member 18 where it terminates at a point proximate the radiating section 16 and includes a thermocouple (not shown in FIG. 1) to detect the temperature of the microwave ablation instrument 10. The entire hub 26, hub cap 28, and transition 12, once assembled, are placed within a handle assembly 46 for ease of gripping and other ergonomic concerns.

The second tubular member 20 has a geometry with an outer diameter 50 that remains substantially constant along a longitudinal axis A of the second tubular member 20. Owing in part to its geometry, the second tubular member 20 may exhibit flexure during operation, for instance, in embodiments where the second tubular member 20 is relatively long (for example, 30 cm). Such flexure reduces and distorts the tactile feedback from the distal tip of the microwave ablation instrument 10 to the handle assembly 46. Although stiffness of the second tubular member 20 may be increased by uniformly increasing the diameter 50 of the second tubular member 20 along an entire length of the second tubular member 20 along the longitudinal axis A, doing so would also result in larger needle track sizes in the target tissue, an increased risk of bleeding and damage to the target tissue, and an increased post-surgery healing time. In embodiments, the various tubular members of the present disclosure have geometries with outer diameters that vary along the longitudinal axes of the tubular members. More particularly, the outer diameters of the tubular members of the present disclosure increase at various rates and at various points along the longitudinal axes thereof in a direction from the distal portion toward the proximal portion. By virtue of their geometries, the various tubular members of the present disclosure exhibit increased stiffness, even for relatively large tubular member lengths, thereby minimizing the flexure of the tubular member during operation and improving the tactile feedback from a distal tip of the instrument to a handle located at a proximal portion of the instrument. At the same time, with relatively small outer diameters at a distal portion, the tubular members of the present disclosure also minimize needle track size, thereby minimizing the risk of bleeding and damage to the target tissue and minimizing post-surgery healing time.

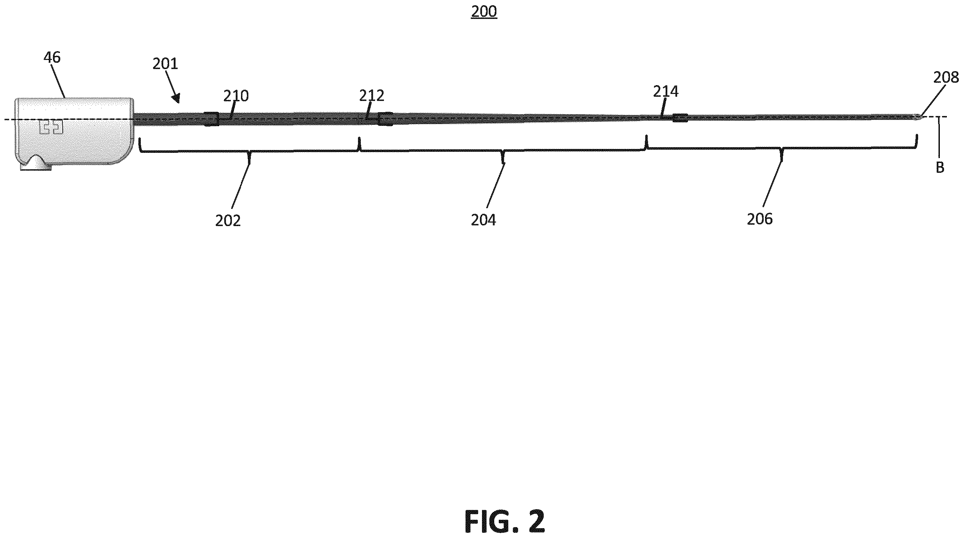

FIG. 2 shows a side view of an illustrative embodiment of a portion of a laparoscopic microwave ablation instrument 200, in accordance with the present disclosure. In embodiments, the laparoscopic microwave ablation instrument 200 of FIG. 2 is similar in several respects to the microwave ablation instrument 10 of FIG. 1, but with several differences. The laparoscopic microwave ablation instrument 200 includes a handle assembly 46 affixed to a tubular member 201. The handle assembly 46 of FIG. 2 is similar to that depicted in FIG. 1. The tubular member 201 includes a proximal portion 202, an intermediate portion 204, and a distal portion 206, with the distal portion 206 being terminated by a distal tip 208. The intermediate portion 204 is interposed between the proximal portion 202 and the distal portion 206. The distal tip 208 includes a needle for piercing tissue during operation.

In contrast to the second tubular member 20 of the instrument 10 (FIG. 1), which has an outer diameter 50 that remains substantially uniform along the longitudinal axis A of the second tubular member 20, the tubular member 201 of the laparoscopic microwave ablation instrument 200 has an outer diameter, 214, 212, 210 that increases at various points along a longitudinal axis B thereof in a direction from the distal portion 206 toward the proximal portion 202. More particularly, in the illustrative embodiment of FIG. 2, the outer diameter 210 remains substantially uniform along the longitudinal axis B throughout the proximal portion 202, and the outer diameter 214 remains substantially uniform along the longitudinal axis B throughout the distal portion 206, with the diameter 214 being smaller than the diameter 210. The outer diameter 212 of the intermediate portion 204 of the tubular member 201 is tapered, increasing in a direction from the distal portion 206 to the proximal portion 202. A maximum of the outer diameter 210 of the tubular member 201 within the proximal portion 202 is greater than a maximum of the outer diameter 212 of the tubular member 201 within the intermediate portion 204; and the maximum of the outer diameter 212 of the tubular member 201 within the intermediate portion 204 is greater than a maximum of the outer diameter 214 of the tubular member 201 within the distal portion 206. In some embodiments, because of the outer diameter of the tubular member 201 increases only in the direction from the distal portion 206 toward the proximal portion 202, not in the direction from the proximal portion 202 toward the distal portion 206, insertion of the tubular member 201 into a patient has a dilating effect.

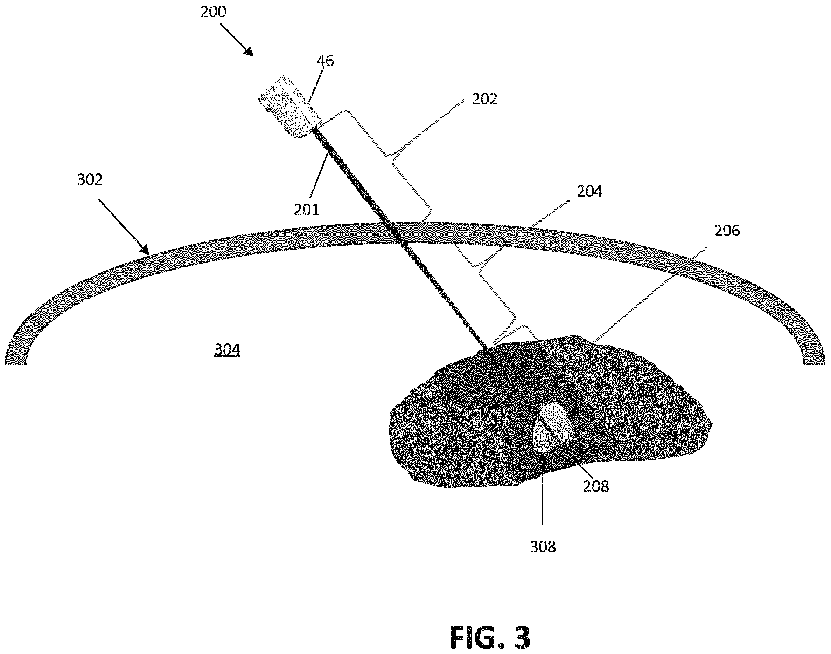

FIG. 3 shows an illustrative embodiment of how the instrument 200 of FIG. 2 may be used to treat a target lesion 308. In embodiments, the respective lengths of the distal portion 206, the intermediate portion 204, and the proximal portion 202 of the instrument 200 are predetermined and configured based on expected dimensions and insertion depth for a surgical procedure. In particular, the lengths of the intermediate portion 204 and/or the proximal portion 202 of the instrument 200 are sized to pass through an incision in the patient skin 302, but to not enter a target organ 306. To minimize needle track size in the target organ 306, the distal portion 206, which generally has an outer diameter that is smaller than the outer diameters in the intermediate portion 204 and the proximal portion 202, is the only portion of the instrument 200 that is sized to penetrate the target organ 306. Thus, invasiveness is minimized at the target organ 306.

Because the patient skin (for instance, the abdominal or thoracic wall) exhibits a lower risk of complication from larger punctures than internal organs, such as the liver, exhibit, invasiveness in the patient skin 302 region may be increased relative to the invasiveness in the insufflated cavity 304 and target organ 306 regions. Thus, in embodiments, the outer diameter of the proximal portion 202 is larger than the outer diameter of the intermediate portion 204, which itself is larger than the outer diameter of the distal portion 206. In some examples, the outer diameter of the proximal portion 202 ranges from 3 to 5 mm. By enlarging the outer diameter of the tubular member 201 within the proximal portion 202, where the patient skin 302 can bear larger punctures, while minimizing the outer diameter of the tubular member 201 within the distal portion 206, where the target organ 306 fares better with a smaller puncture, the overall stiffness of the tubular member 201 is increased while minimizing the invasiveness at the target organ 306.

In embodiments, a length of the distal portion 206 is predetermined based on an expected insertion depth of the distal portion 206 into the target organ 306, such as a portion of a particular internal organ, or target lesion 308. For example, for a laparotomy or a liver ablation procedure, between approximately 10 cm and 20 cm of the tubular member 201 is expected to be inserted into the liver to reach target tissue. Therefore, in some embodiments, the length of the distal portion 206 may range from 10 cm to 20 cm. A length of the intermediate portion 204 is predetermined based on an expected insertion depth of the intermediate portion 204 into the abdominal cavity 304 of the patient. The length of the proximal portion 202, which is the portion of the tubular member 201 having the largest diameter, is predetermined to provide sufficient stiffness of the tubular member 201 and maneuverability of the instrument 200. In some examples, the length of the proximal portion 202 is approximately 10 cm.

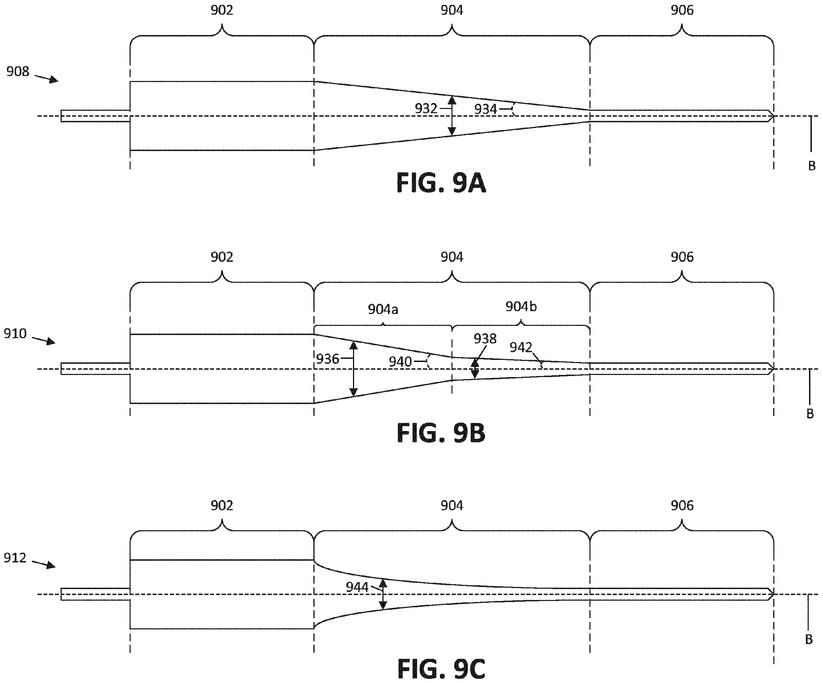

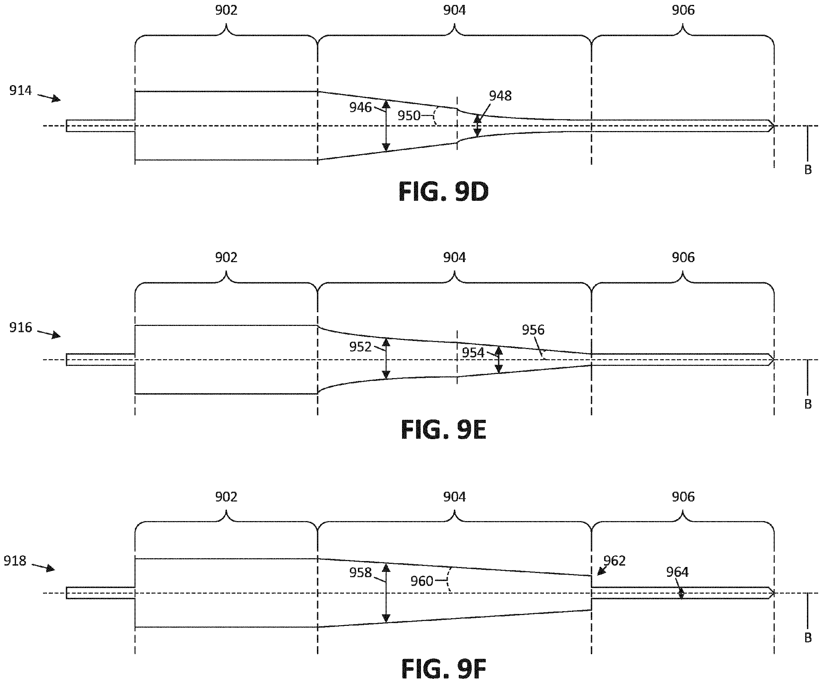

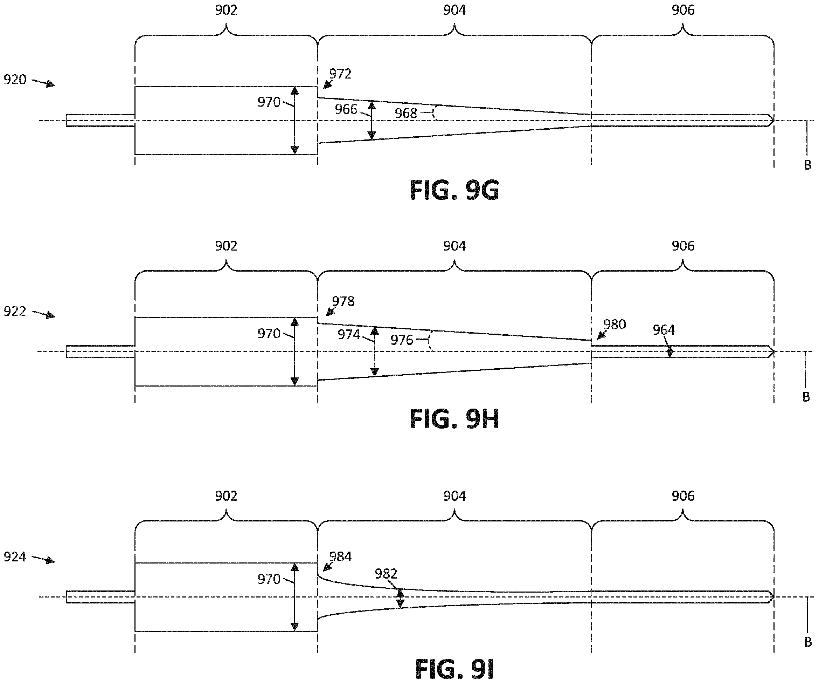

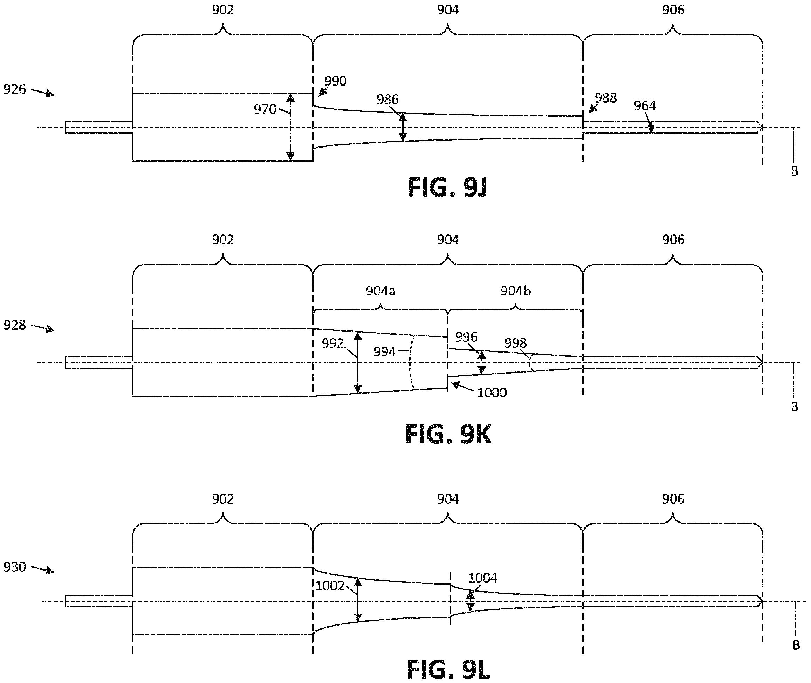

For a tubular member, such as the second tubular member 20 (FIG. 1), having an outer diameter that remains essentially constant along a longitudinal axis thereof, the force required to deflect the tip when the fulcrum (for instance, the patient skin or abdominal wall) is located near the distal tip is relatively high, and the force required to deflect the tip when the fulcrum is located near the handle assembly 46 is relatively low. In accordance with the present disclosure, if the tubular member 201 is formed with a geometric profile that is optimized to maintain the force required to deflect the tip as a function of distance between the fulcrum and the tip, then the deflection response is flatter despite different fulcrum locations. In other words, the force required to deflect the tip remains closer to constant regardless of where the fulcrum is located, and tactile feedback is transferred from the distal portion 206 of the tubular member 201 to the proximal portion 202, so the user feels the tissue response during insertion into the target tissue 306. To transition the profile of the smaller outer diameter of the tubular member 201 at the distal portion 206 to the larger outer diameter of the tubular member 201 at the proximal portion 202, a derivation may be used. In particular, the derivation is employed to determine the geometry of the outer diameter of tubular member 201 within the intermediate portion 204, more specifically how the outer diameter thereof increases along the longitudinal axis B, in a direction from the distal portion 206 to the proximal portion 202, in a manner which counters loss of stiffness (or bendability) with distance from the distal tip 208. In the illustrative embodiment of the laparoscopic microwave ablation instrument 200 illustrated in FIGS. 2 and 3, the outer diameter of the tubular member 201 within the intermediate portion 204 increases linearly along the longitudinal axis B, in a direction from the distal portion 206 to the proximal portion 202. However, other geometries of the tubular member 201 are also contemplated, for instance, as described in further detail below with reference to FIGS. 9A through 9L.

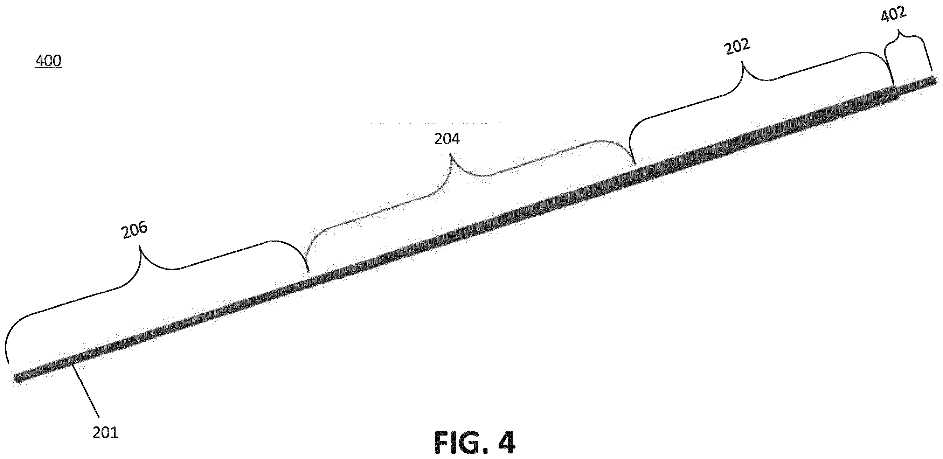

Having described certain aspects of the laparoscopic microwave ablation instrument 200 and how the instrument 200 may be used to treat the target lesion 308 in connection with FIGS. 2 and 3, reference is now made to FIGS. 4 through 8 to describe additional aspects of the instrument 200. FIG. 4 shows a perspective view 400 of the tubular member 201 of the laparoscopic microwave ablation instrument 200 of FIG. 2, with all other components of the instrument 200 removed for clarity. Although not shown in FIG. 4, the tubular member 201 is configured to mate with a hub cap (for instance, the hub cap 28 of FIG. 1) at a proximal end thereof. In some embodiments, the tubular member 201 includes a portion 402 that is located adjacent to the proximal portion 202 and has an outer diameter that is smaller than the outer diameter of the proximal portion 202 in order to fit within a cavity of the hub cap 28 and handle assembly 46 that, in other assemblies, can also accommodate the second tubular member 20 of FIG. 1. The outer diameter of the portion 402, for example, may be the same as the outer diameter of the distal portion 206. Although not shown in FIG. 4, in other embodiments, the outer diameter of the portion 402 is greater than or equivalent to the maximum outer diameter of the proximal portion 202, and is configured to fit within a cavity of a hub cap and handle assembly that are larger than the hub cap 28 and handle assembly 46 depicted in FIG. 1.

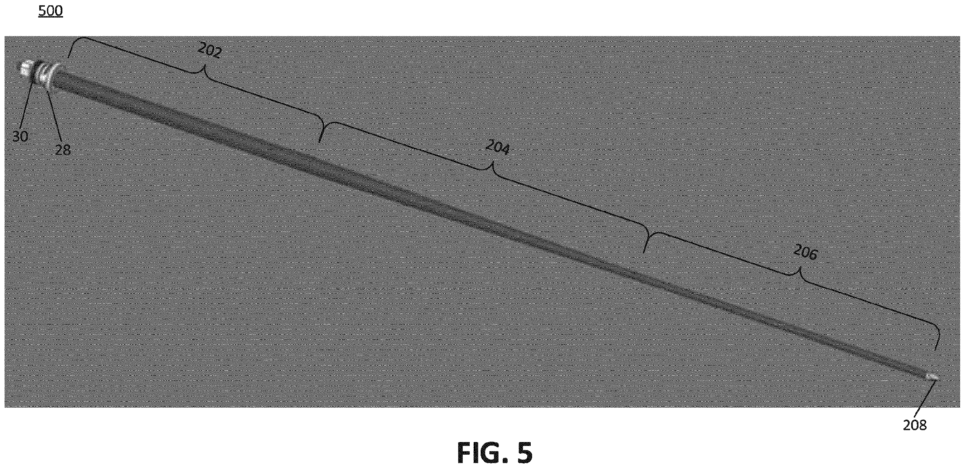

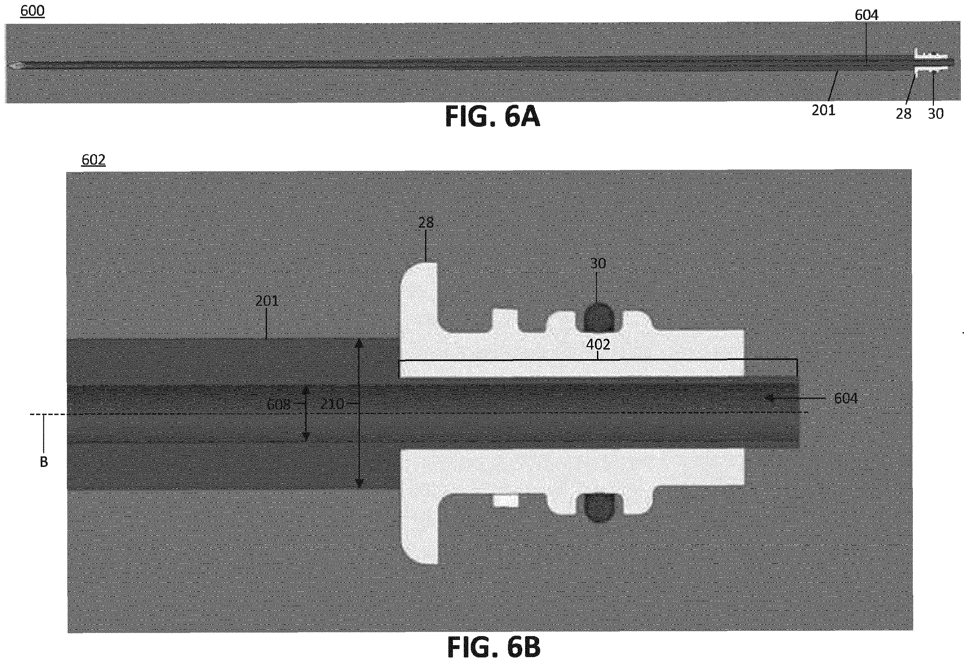

FIG. 5 shows a perspective view 500 of the tubular member 201 of FIG. 4 shown with the hub cap 28 coupled to the portion 402 (not visible in FIG. 5) and with the o-ring 30 arranged around the hub cap 28. FIG. 6A and FIG. 6B show side cross-sectional views 600 and 602, respectively, of the tubular member 201, the hub cap 28, and the o-ring 30 of FIG. 5. The proximal portion 202, the intermediate portion 204, and the distal portion 206 of the tubular member 201 form a channel 604 therethrough.

In designing the geometries of the outer and inner diameters of the tubular member 201 along its longitudinal axis B, various factors may be considered. In particular, the area moment of inertia of a cross-section of a beam is a property used to calculate a beam's deflection and resulting stresses caused by bending that beam. The area moment of inertia of a cylindrical tube ($I_{tube}$) is defined by Equation 1 below, where $OD$ represents the outer diameter of the tube and $ID$ represents the inner diameter of the tube.

$$(1) \qquad I_{tube} = \frac{\pi(OD^4 - ID^4)}{64}$$

The bending stiffness ($K$) is the resistance of the tube to bending defined by Equation 2 below, where $P$ represents the applied force and $W$ represents an amount of deflection.

$$(2) \qquad K = \frac{P}{W}$$

The bending stiffness ($K$) is a function of the elastic modulus ($E_{tube}$) of the tube, the area moment of inertia of the tube ($I_{tube}$), the length of the tube ($L$) and the boundary/loading condition of the tube. The relationship between the applied moment $M$ (force over a length) and the change in deflection of the tube is given by Equation 3 below.

$$(3) \qquad M = E_{tube} \times I_{tube} \times \frac{d^2w}{dx^2}$$

Substituting for $I_{tube}$ in Equation 3 yields Equation 4 below.

$$(4) \qquad M = E_{tube} \times \frac{\pi(OD^4 - ID^4)}{64} \times \frac{d^2w}{dx^2}$$

Although increasing the inner diameter may not be as effective, a small increase in the outer diameter ($OD$) of the majority of the tube leads to a large increase in overall bending moment, in other words, a stiffer tube. This increase in stiffness enables the movement imparted by the user at the handle to be more efficiently translated to the movement of the distal tip, which, as noted above, is particularly useful in laparoscopic surgical procedures.

Accordingly, with continued reference to FIG. 6A and FIG. 6B, the outer diameter of the tubular member 201 varies along the longitudinal axis B thereof (for instance, with the outer diameter 210 of the proximal portion 202 being larger than the outer diameter 214 (FIG. 2) of the distal portion 206), thereby providing a stiffer tubular member 201 than would result otherwise. The proximal portion 202, the intermediate portion 204, and the distal portion 206 of the tubular member 201 have an equivalent inner diameter 608 that remains uniform along the longitudinal axis B of the tubular member 201. The view 602 also shows the portion 402 of the tubular member 201 inserted into the cavity of the hub cap 28. The portion 402 has an outer diameter that is smaller than the outer diameter 210 of the proximal portion 202, and has an inner diameter that is equivalent to the inner diameter 608 of the proximal portion 202, the intermediate portion 204, and the distal portion 206.

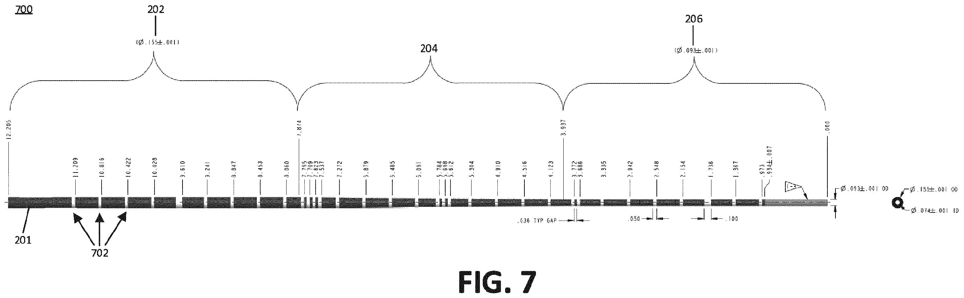

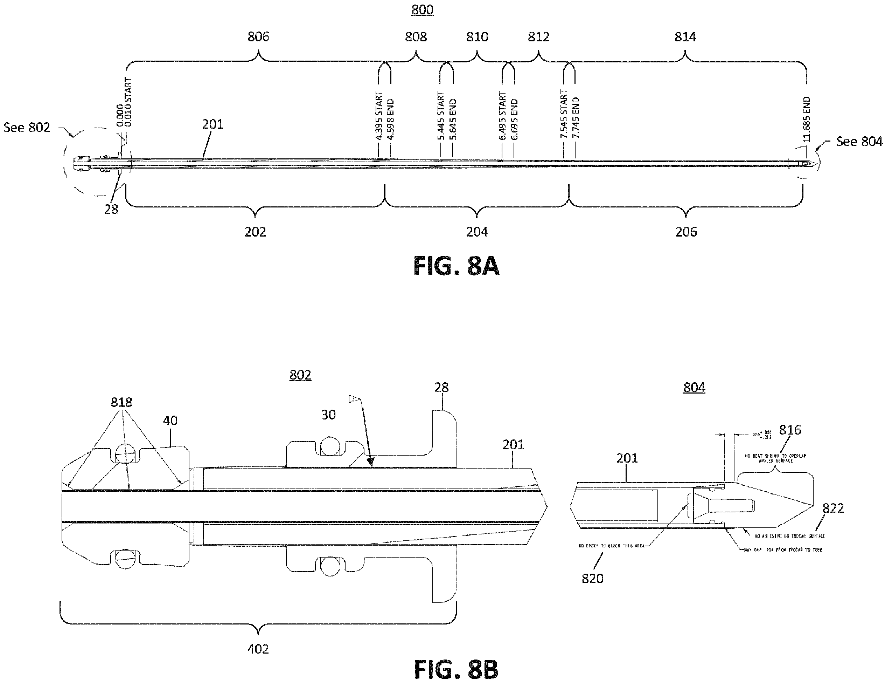

FIGS. 7, 8A, and 8B include additional views 700, 800, 802, and 804 of the tubular member 201, hub cap 28, and o-ring 30 of FIG. 6A and FIG. 6B, showing additional details regarding the construction thereof. In some embodiments, the tubular member 201 may be formed of fiberglass, steel, ceramic, sapphire, carbon fiber, composite blends, or any other suitable material. In another embodiment, the tubular member 201 may be formed of shape memory fibers that form a structural weave within a non-conductive substrate. The tubular member 201, in some examples, may be constructed by first performing a pultrusion process, whereby material is pulled through a die resulting in a preliminary tubular member with a uniform cross section (having an outer diameter that matches the maximum outer diameter desired of the finally constructed tubular member 201), and then grinding various portions of the preliminary tubular member to have the desired outer diameter (for instance, grinding the intermediate portion 204 and the distal portion 206 to have the outer diameters 212 and 214, respectively, as described and shown in connection with FIG. 2, and grinding the portion 402 to have the outer diameter shown in FIG. 4).

In some embodiments, after the tubular member 201 has been formed by pultrusion and grinding, depth indicators 702 are pad printed on an outer surface of the tubular member 201 to indicate to the user, during a surgical procedure, a depth to which the tubular member 201 is inserted into the patient. FIG. 7 is a side view 700 of the tubular member 201 of FIG. 4, showing an illustrative embodiment of the depth indicators 702 that may be included thereon. The depth indicators 702, in some examples, may be pad printed at predetermined locations along the tubular member 201 using a color (for instance, white) that is different from the color of the remainder of the tubular member 201. The depth indicators 702 may be evenly distributed along a longitudinal axis B of the tubular member 201. In some instances, the depth indicators 702 may be grouped together at various portions of the tubular member 201, for instance, near boundaries between the proximal portion 202, the intermediate portion 204, and the distal portion 206, to aid the user in quickly determining which portion(s) of the tubular member 201 are within the patient.

After the depth indicators 702 have been printed on the outer surface of the tubular member 201, multiple sizes of heatshrink material 806, 808, 810, 812, 814 are adhered to the outer surface of the tubular member 201. In order to match the geometry of the tubular member 201, each of the heatshrink materials 806, 808, 810, 812, 814 has a particular pre-shrunken diameter and shrunken diameter, and is affixed to a corresponding portion of the tubular member 201 as depicted in view 800. As shown in view 804, heatshrink material is omitted from the angled surface 816. As shown in view 802, hub divider 40 is bonded to portion 402 of the tubular member 201 at locations 818 using epoxy or any suitable adhesive, and area 820 and trocar surface 822 remain unblocked by any epoxy or adhesive.

The outer diameter of the tubular member 201 described and shown in connection with FIGS. 2 through 8 increases in a linear manner within the intermediate portion 204, along the longitudinal axis B, in a direction from the distal portion 206 to the proximal portion 202. However, other geometries of tubular members are also contemplated, for instance, where the outer diameter of the tubular member increases in a variety of particular manners (linearly, exponentially, at particular linear or exponential growth rates, gradually or with steps for tactile feedback effect, and/or the like) within the intermediate portion along its longitudinal axis B, in a direction from the distal portion to the proximal portion. FIGS. 9A through 9L are side views of additional illustrative embodiments of laparoscopic microwave instrument tubular members 908 through 930. The geometries of the proximal portions 902 and distal portions 906 of the tubular members 908 through 930 are similar to one another. However, each of the tubular members 908 through 930 has a particular geometry of the intermediate portion 904. It should be understood that, while the tubular members 908 through 930 are shown and described for illustrative purposes, other combination of the aspects of the tubular members 908 through 930 are also contemplated.

The intermediate portion 904 of the tubular member 908 has an outer diameter 932 that increases linearly, at a uniform angle 934 with respect to the longitudinal axis B, in a direction from the distal portion 906 toward the proximal portion 902.

The intermediate portion 904 of the tubular member 910 includes multiple subportions 904a and 904b. The subportions 904a and 904b of the tubular member 910 have outer diameters 936 and 938, respectively, that increase at angles 940 and 942, respectively, with respect to the longitudinal axis B, in a direction from the distal portion 906 toward the proximal portion 902.

The intermediate portion 904 of the tubular member 912 has an outer diameter 944 that increases exponentially in a direction from the distal portion 906 toward the proximal portion 902.

The intermediate portion 904 of the tubular member 914 includes multiple subportions 904a and 904b. The subportion 904a of the tubular member 914 has an outer diameter 946 that increases linearly, at an angle 950 with respect to the longitudinal axis B, in a direction from the distal portion 906 toward the proximal portion 902. The subportion 904b of the tubular member 914 has an outer diameter 948 that increases exponentially in the direction from the distal portion 906 toward the proximal portion 902. Although not shown in FIG. 9D, in some embodiments, near a junction between the subportion 904a and the subportion 904b of the tubular member 914, the outer diameter 946 of the subportion 904a is greater than the outer diameter 948 of the subportion 904b, thus forming a step at the junction between the subportion 904a and the subportion 904b.

The intermediate portion 904 of the tubular member 916 includes multiple subportions 904a and 904b. The subportion 904a of the tubular member 916 has an outer diameter 952 that increases exponentially in the direction from the distal portion 906 toward the proximal portion 902. The subportion 904b of the tubular member 916 has an outer diameter 954 that increases linearly, at an angle 956 with respect to the longitudinal axis B, in a direction from the distal portion 906 toward the proximal portion 902. Although not shown in FIG. 9E, in some embodiments, near a junction between the subportion 904a and the subportion 904b of the tubular member 916, the outer diameter 952 of the subportion 904a is greater than the outer diameter 954 of the subportion 904b, thus forming a step at the junction between the subportion 904a and the subportion 904b.

The intermediate portion 904 of the tubular member 918 has an outer diameter 958 that increases linearly, at an angle 960 with respect to the longitudinal axis B, in a direction from the distal portion 906 toward the proximal portion 902. Additionally, near a junction between the distal portion 906 and the intermediate portion 904, the outer diameter 958 of the intermediate portion 904 is greater than an outer diameter 964 of the distal portion 906, thus forming a step 962 at the junction between the distal portion 906 and the intermediate portion 904.

The intermediate portion 904 of the tubular member 920 has an outer diameter 966 that increases linearly, at an angle 968 with respect to the longitudinal axis B, in a direction from the distal portion 906 toward the proximal portion 902. Additionally, near a junction between the intermediate portion 904 and the proximal portion 902, an outer diameter 970 of the proximal portion 902 is greater than the outer diameter 966 of the intermediate portion 904, thus forming a step 972 at the junction between the intermediate portion 904 and the proximal portion 902.

The intermediate portion 904 of the tubular member 922 has an outer diameter 974 that increases linearly, at an angle 976 with respect to the longitudinal axis B, in a direction from the distal portion 906 toward the proximal portion 902. Additionally, near a junction between the intermediate portion 904 and the proximal portion 902, the outer diameter 970 of the proximal portion 902 is greater than the outer diameter 974 of the intermediate portion 904, thus forming a step 978 at the junction between the intermediate portion 904 and the proximal portion 902. Additionally, near a junction between the distal portion 906 and the intermediate portion 904, the outer diameter 974 of the intermediate portion 904 is greater than the outer diameter 964 of the distal portion 906, thus forming a step 980 at the junction between the distal portion 906 and the intermediate portion 904.

The intermediate portion 904 of the tubular member 924 has an outer diameter 982 that increases exponentially in a direction from the distal portion 906 toward the proximal portion 902. Additionally, near a junction between the proximal portion 902 and the intermediate portion 904 of the tubular member 924, the outer diameter 970 of the proximal portion 902 is greater than the outer diameter 982 of the intermediate portion 904, thus forming a step 984 at the junction between the proximal portion 902 and the intermediate portion 904.

The intermediate portion 904 of the tubular member 926 has an outer diameter 986 that increases exponentially in a direction from the distal portion 906 toward the proximal portion 902. Near a junction between the distal portion 906 and the intermediate portion 904, the outer diameter 986 of the intermediate portion 904 is greater than the outer diameter 964 of the distal portion 906, thus forming a step 988 at the junction between the distal portion 906 and the intermediate portion 904. Additionally, near a junction between the proximal portion 902 and the intermediate portion 904, the outer diameter 970 of the proximal portion 902 is greater than the outer diameter 986 of the intermediate portion 904, thus forming a step 990 at the junction between the proximal portion 902 and the intermediate portion 904.

The intermediate portion 904 of the tubular member 928 includes multiple subportions 904a and 904b. The subportion 904a of the tubular member 928 has an outer diameter 992 that increases linearly, at an angle 994 with respect to the longitudinal axis B, in the direction from the distal portion 906 toward the proximal portion 902. The subportion 904b of the tubular member 928 has an outer diameter 996 that increases linearly, at an angle 998 with respect to the longitudinal axis B, in a direction from the distal portion 906 toward the proximal portion 902. Additionally, near a junction between the subportion 904a and the subportion 904b of the tubular member 916, the outer diameter 992 of the subportion 904a is greater than the outer diameter 996 of the subportion 904b, thus forming a step 1000 at the junction between the subportion 904a and the subportion 904b.

The intermediate portion 904 of the tubular member 930 includes multiple subportions 904a and 904b. The subportion 904a of the tubular member 928 has an outer diameter 1002 that increases exponentially, at a corresponding growth rate, with respect to the longitudinal axis B, in the direction from the distal portion 906 toward the proximal portion 902. The subportion 904a of the tubular member 930 has an outer diameter 1004 that increases exponentially, at a corresponding growth rate (which may be equivalent to, or unequal to, the growth rate at which the outer diameter 1002 of the subportion 904a grows), with respect to the longitudinal axis B, in the direction from the distal portion 906 toward the proximal portion 902.

In addition to the embodiments shown in FIGS. 9A through 9L, tubular members in some embodiments may have multiple subportions, each having a corresponding length, respectively, which lengths may be equivalent to one another or unequal to one another. Additionally, each of the subportions may have a corresponding outer diameter that increases at a respective linear or exponential rate in a direction from the distal portion 902 toward the proximal portion 906.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to similar or identical elements throughout the description of the figures.

The foregoing description is only illustrative of the present laparoscopic microwave ablation instrument tubular members. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

**Claims**

1. A laparoscopic microwave ablation instrument, comprising a handle assembly (46) affixed to a tubular member (201, 908, 910, 912, 914, 916, 918, 920, 922, 924, 926, 928, 930), wherein the tubular member comprises:

   a proximal portion (202, 902);

a distal portion (206, 906), wherein the distal portion is terminated by a distal tip (208) forming a needle for piercing tissue during operation; and an intermediate portion (204, 904) interposed between the proximal portion and the distal portion,

wherein a maximum outer diameter (210, 970) of the proximal portion is greater than or equal to a maximum outer diameter (212) of the intermediate portion,

wherein the maximum outer diameter (212) of the intermediate portion is greater than or equal to a maximum outer diameter (214, 964) of the distal portion, and

a microwave transmission line having a radiating portion, wherein the microwave transmission line is inserted within the tubular member, wherein:

the maximum outer diameter (210, 970) of the proximal portion (202) is greater than the maximum outer diameter (214, 964) of the distal portion (206); and

the intermediate portion (204, 904) has an outer diameter at least a portion of which increases in a direction from the distal portion (206, 906) to the proximal portion (202, 902).

2. The laparoscopic microwave ablation instrument of claim 1,
wherein the proximal portion (202, 902), the distal portion (206, 906), and the intermediate portion (204, 904) of the tubular member are each tubular, and
wherein the proximal portion, the distal portion, and the intermediate portion each have an equivalent inner diameter that remains uniform along a longitudinal axis of the tubular member.

3. The laparoscopic microwave ablation instrument of claim 1 or 2, wherein the outer diameter (214) of the distal portion (206) of the tubular member (201) remains uniform along a longitudinal axis of the distal portion.

4. The laparoscopic microwave ablation instrument of claim 3, wherein the outer diameter (210, 970) of the proximal portion (202, 902) of the tubular member remains uniform along a longitudinal axis of the proximal portion.

5. The laparoscopic microwave ablation instrument of any preceding claim, wherein the intermediate portion (904) of the tubular member (908, 918, 920, 922) has an outer diameter (932, 958, 966, 974) that increases linearly in a direction from the distal portion (906) toward the proximal portion (902).

6. The laparoscopic microwave ablation instrument of claim 1,
wherein the intermediate portion (904) of the tubular member (910, 928) includes a plurality of subportions (904a, 904b), and
wherein each of the plurality of subportions has an outer diameter (936, 938, 992, 996) that increases linearly, at a respective angle (994, 998), in a direction from the distal portion (906) toward the proximal portion (902).

7. The laparoscopic microwave ablation instrument of claim 1, wherein the intermediate portion (904) of the tubular member (912, 924, 926) has an outer diameter that increases exponentially in a direction from the distal portion (906) toward the proximal portion (902).

8. The laparoscopic microwave ablation instrument of claim 1,
wherein the intermediate portion (904) of the tubular member (930) includes a plurality of subportions, and
wherein each of the plurality of subportions has an outer diameter (1002, 1004) that increases exponentially, at a respective growth rate, in a direction from the distal portion (906) toward the proximal portion (902).

9. The laparoscopic microwave ablation instrument of claim 1,
wherein the intermediate portion of the tubular member (914, 916) includes a first subportion and a second subportion,
wherein the first subportion has an outer diameter (946, 954) that increases linearly in a direction from the distal portion (906) toward the proximal portion (902), and
wherein the second subportion has an outer diameter (948, 952) that increases exponentially in the direction from the distal portion (906) toward the proximal portion (902).

10. The laparoscopic microwave ablation instrument of claim 9, wherein the first subportion and the second subportion are arranged in a direction from the distal portion (906) to the proximal portion (902).

11. The laparoscopic microwave ablation instrument of claim 10, wherein, near a junction between the first subportion and the second subportion of the tubular member, the outer diameter of the second subportion is greater than the outer diameter of the first subportion, thus forming a step at the junction between the first subportion and the second subportion.

12. The laparoscopic microwave ablation instrument of claim 8, wherein the first subportion and the second subportion of the tubular member are arranged in a direction from the proximal portion to the distal portion; preferably wherein, near a junction between the first subportion and the second subportion, the outer diameter of the first subportion is greater than the outer diameter of the second subportion, thus forming a step at the junction between the first subportion and the second subportion.

13. The laparoscopic microwave ablation instrument of any preceding claim, wherein the intermediate portion (904) of the tubular member (922) has an outer diameter (974) that increases linearly in a direction from the distal portion (906) toward the proximal portion (902), and wherein, near a junction between the distal portion and the intermediate portion, the outer diameter of the intermediate portion is greater than an outer diameter of the distal portion, thus forming a step (980) at the junction between the distal portion and the intermediate portion; preferably wherein, near a junction between the intermediate portion (904) and the proximal portion (902), an outer diameter of the proximal portion is greater than the outer diameter of the intermediate portion, thus forming a step (978) at the junction between the intermediate portion and the proximal portion.

14. The laparoscopic microwave ablation instrument of any preceding claim, wherein the intermediate portion (904) of the tubular member (926) has an outer diameter (986) that increases exponentially in a direction from the distal portion (906) toward the proximal portion (902), and wherein, near a junction between the distal portion and the intermediate portion, the outer diameter of the intermediate portion is greater than an outer diameter of the distal portion, thus forming a step (988) at the junction between the distal portion and the intermediate portion.

15. The laparoscopic microwave ablation instrument of claim 14, wherein, near a junction between the intermediate portion (904) and the proximal portion (902) of the tubular member, an outer diameter (970) of the proximal portion is greater than the outer diameter (986) of the intermediate portion, thus forming a step (990) at the junction between the intermediate portion and the proximal portion.

**Patentansprüche**

1. Laparoskopisches Mikrowellenablationsinstrument, umfassend eine Griffanordnung (46), die an einem röhrenförmigen Element (201, 908, 910, 912, 914, 916, 918, 920, 922, 924, 926, 928, 930) befestigt ist, wobei das röhrenförmige Element umfasst:

   einen proximalen Abschnitt (202, 902);

einen distalen Abschnitt (206, 906), wobei der distale Abschnitt durch eine distale Spitze (208) abgeschlossen ist, die eine Nadel zum Durchstechen von Gewebe während einer Operation bildet; und

einen Zwischenabschnitt (204, 904), der zwischen dem proximalen Abschnitt und dem distalen Abschnitt angeordnet ist, wobei ein maximaler Außendurchmesser (210, 970) des proximalen Abschnitts größer gleich einem maximalen Außendurchmesser (212) des Zwischenabschnitts ist, wobei der maximale Außendurchmesser (212) des Zwischenabschnitts größer gleich einem maximalen Außendurchmesser (214, 964) des distalen Abschnitts ist, und eine Mikrowellenübertragungsleitung mit einem Strahlungsabschnitt, wobei die Mikrowellenübertragungsleitung in das röhrenförmige Element eingesetzt ist, wobei:

   der maximale Außendurchmesser (210, 970) des proximalen Abschnitts (202) größer als der maximale Außendurchmesser (214, 964) des distalen Abschnitts (206) ist; und
   der Zwischenabschnitt (204, 904) einen Außendurchmesser aufweist, von dem mindestens ein Abschnitt in einer Richtung vom distalen Abschnitt (206, 906) zum proximalen Abschnitt (202, 902) zunimmt.

2. Laparoskopisches Mikrowellenablationsinstrument nach Anspruch 1, wobei der proximale Abschnitt (202, 902), der distale Abschnitt (206, 906) und der Zwischenabschnitt (204, 904) des röhrenförmigen Elements jeweils röhrenförmig sind, und wobei der proximale Abschnitt, der distale Abschnitt und der Zwischenabschnitt jeweils einen äquivalenten Innendurchmesser aufweisen, der entlang einer Längsachse des rohrförmigen Elements gleichmäßig bleibt.

3. Laparoskopisches Mikrowellenablationsinstrument nach Anspruch 1 oder 2, wobei der Außendurchmesser (214) des distalen Abschnitts (206) des röhrenförmigen Elements (201) entlang einer Längsachse des distalen Abschnitts gleichmäßig bleibt.

4. Laparoskopisches Mikrowellenablationsinstrument nach Anspruch 3, wobei der Außendurchmesser (210, 970) des proximalen Abschnitts (202, 902) des röhrenförmigen Elements entlang einer Längsachse des proximalen Abschnitts gleichmäßig bleibt.

5. Laparoskopisches Mikrowellenablationsinstrument nach einem vorhergehenden Anspruch, wobei der Zwischenabschnitt (904) des röhrenförmigen Elements (908, 918, 920, 922) einen Außendurchmesser (932, 958, 966, 974) aufweist, der in einer Richtung vom distalen Abschnitt (906) hin zum proximalen Abschnitt (902) linear zunimmt.

6. Laparoskopisches Mikrowellenablationsinstrument nach Anspruch 1, wobei der Zwischenabschnitt (904) des röhrenförmigen Elements (910, 928) mehrere Unterabschnitte (904a, 904b) enthält und wobei jeder der mehreren Unterabschnitte einen Außendurchmesser (936, 938, 992, 996) aufweist, der in einem jeweiligen Winkel (994, 998) in einer Richtung vom distalen Abschnitt (906) hin zum proximalen Abschnitt (902) linear zunimmt.

7. Laparoskopisches Mikrowellenablationsinstrument nach Anspruch 1, wobei der Zwischenabschnitt (904) des röhrenförmigen Elements (912, 924, 926) einen Außendurchmesser aufweist, der in einer Richtung vom distalen Abschnitt (906) hin zum proximalen Abschnitt (902) exponentiell zunimmt.

8. Laparoskopisches Mikrowellenablationsinstrument nach Anspruch 1, wobei der Zwischenabschnitt (904) des röhrenförmigen Elements (930) mehrere Teilabschnitte enthält, und wobei jeder der mehreren Teilabschnitte einen Außendurchmesser (1002, 1004) aufweist, der mit einer jeweiligen Wachstumsrate in einer Richtung vom distalen Abschnitt (906) hin zum proximalen Abschnitt (902) exponentiell zunimmt.

9. Laparoskopisches Mikrowellenablationsinstrument nach Anspruch 1, wobei der Zwischenabschnitt des röhrenförmigen Elements (914, 916) einen ersten Teilabschnitt und einen zweiten Teilabschnitt enthält, wobei der erste Teilabschnitt einen Außendurchmesser (946, 954) aufweist, der in einer Richtung vom distalen Abschnitt (906) hin zum proximalen Abschnitt (902) linear zunimmt, und wobei der zweite Teilabschnitt einen Außendurchmesser (948, 952) aufweist, der in der Richtung vom distalen Abschnitt (906) hin zum proximalen Abschnitt (902) exponentiell zunimmt.

10. Laparoskopisches Mikrowellenablationsinstrument nach Anspruch 9, wobei der erste Teilabschnitt und der zweite Teilabschnitt in einer Richtung vom distalen Teil (906) zum proximalen Teil (902) angeordnet sind.

11. Laparoskopisches Mikrowellenablationsinstrument nach Anspruch 10, wobei der Außendurchmesser des zweiten Teilabschnitts nahe einer Verbindungsstelle zwischen dem ersten Teilabschnitt und dem zweiten Teilabschnitt des röhrenförmigen Elements größer als der Außendurchmesser des ersten Teilabschnitts ist, wodurch an der Verbindungsstelle zwischen dem ersten Teilabschnitt und dem zweiten Teilabschnitt eine Stufe gebildet wird.

12. Laparoskopisches Mikrowellenablationsinstrument nach Anspruch 8, wobei der erste Teilabschnitt und der zweite Teilabschnitt des röhrenförmigen Elements in einer Richtung vom proximalen Abschnitt zum distalen Abschnitt angeordnet sind; vorzugsweise wobei der Außendurchmesser des ersten Teilabschnitts nahe einer Verbindungsstelle zwischen dem ersten Teilabschnitt und dem zweiten Teilabschnitt größer als der Außendurchmesser des zweiten Teilabschnitts ist, wodurch an der Verbindungsstelle zwischen dem ersten Teilabschnitt und dem zweiten Teilabschnitt eine Stufe gebildet wird.

13. Laparoskopisches Mikrowellenablationsinstrument nach einem vorhergehenden Anspruch, wobei der Zwischenabschnitt (904) des röhrenförmigen Elements (922) einen Außendurchmesser (974) aufweist, der in einer Richtung vom distalen Abschnitt (906) hin zum proximalen Abschnitt (902) linear zunimmt, und wobei der Außendurchmesser des Zwischenabschnitts nahe einer Verbindungsstelle zwischen dem distalen Abschnitt und dem Zwischenabschnitt größer als ein Außendurchmesser des distalen Abschnitts ist, wodurch an der Verbindungsstelle zwischen dem distalen Abschnitt und dem Zwischenabschnitt eine Stufe (980) gebildet wird; vorzugsweise wobei ein Außendurchmesser des proximalen Abschnitts nahe einer Verbindungsstelle zwischen dem Zwischenabschnitt (904) und dem proximalen Abschnitt (902) größer als der Außendurchmesser des Zwischenabschnitts ist, wodurch an der Verbindungsstelle zwischen dem Zwischenabschnitt und dem proximalen Abschnitt eine Stufe (978) gebildet wird.

14. Laparoskopisches Mikrowellenablationsinstrument nach einem vorhergehenden Anspruch, wobei der Zwischenabschnitt (904) des röhrenförmigen Elements (926) einen Außendurchmesser (986) aufweist, der in einer Richtung vom distalen Abschnitt (906) hin zum proximalen Abschnitt (902) exponentiell zunimmt, und wobei der Außendurchmesser des Zwischenabschnitts nahe einer Verbindungsstelle zwischen dem distalen Abschnitt und dem Zwischenabschnitt größer als ein Außendurchmesser des distalen Abschnitts ist, wodurch an der Verbindungsstelle zwischen dem distalen Abschnitt und dem Zwischenabschnitt eine Stufe (988) gebildet wird.

15. Laparoskopisches Mikrowellenablationsinstrument nach Anspruch 14, wobei ein Außendurchmesser (970) des proximalen Abschnitts nahe einer Verbindungsstelle zwischen dem Zwischenabschnitt (904) und dem proximalen Abschnitt (902) des röhrenförmigen Elements größer als der Außendurchmesser (986) des Zwischenabschnitts ist, wodurch an der Verbindungsstelle zwischen dem Zwischenabschnitt und dem proximalen Abschnitt eine Stufe (990) gebildet wird.

## Revendications

**1.** Instrument d'ablation laparoscopique par micro-ondes, comprenant un ensemble poignée (46) fixé à un élément tubulaire (201, 908, 910, 912, 914, 916, 918, 920, 922, 924, 926, 928, 930), l'élément tubulaire comprenant :

une partie proximale (202, 902) ;
une partie distale (206, 906), la partie distale étant terminée par une pointe distale (208) formant une aiguille servant à percer le tissu pendant le fonctionnement ; et
une partie intermédiaire (204, 904) interposée entre la partie proximale et la partie distale, un diamètre extérieur maximal (210, 970) de la partie proximale étant supérieur ou égal à un diamètre extérieur maximal (212) de la partie intermédiaire, le diamètre extérieur maximal (212) de la partie intermédiaire étant supérieur ou égal à un diamètre extérieur maximal (214, 964) de la partie distale, et une ligne de transmission de micro-ondes présentant une partie rayonnante, la ligne de transmission de micro-ondes étant insérée à l'intérieur l'élément tubulaire, dans lequel :

le diamètre extérieur maximal (210, 970) de la partie proximale (202) est supérieur au diamètre extérieur maximal (214, 964) de la partie distale (206) ; et
la partie intermédiaire (204, 904) a un diamètre extérieur dont au moins une partie augmente dans une direction allant de la partie distale (206, 906) à la partie proximale (202, 902).

**2.** Instrument d'ablation laparoscopique par micro-ondes selon la revendication 1, dans lequel la partie proximale (202, 902), la partie distale (206, 906) et la partie intermédiaire (204, 904) de l'élément tubulaire sont chacune tubulaires, et dans lequel la partie proximale, la partie distale et la partie intermédiaire ont chacune un diamètre intérieur équivalent qui reste uniforme le long d'un axe longitudinal de l'élément tubulaire.

**3.** Instrument d'ablation laparoscopique par micro-ondes selon la revendication 1 ou 2, dans lequel le diamètre extérieur (214) de la partie distale (206) de l'élément tubulaire (201) reste uniforme le long d'un axe longitudinal de la partie distale.

**4.** Instrument d'ablation laparoscopique par micro-ondes selon la revendication 3, dans lequel le diamètre extérieur (210, 970) de la partie proximale (202, 902) de l'élément tubulaire reste uniforme le long d'un axe longitudinal de la partie proximale.

**5.** Instrument d'ablation laparoscopique par micro-ondes selon l'une quelconque des revendications précédentes, dans lequel la partie intermédiaire (904) de l'élément tubulaire (908, 918, 920, 922) a un diamètre extérieur (932, 958, 966, 974) qui augmente de façon linéaire dans une direction allant de la partie distale (906) vers la partie proximale (902).

**6.** Instrument d'ablation laparoscopique par micro-ondes selon la revendication 1, dans lequel la partie intermédiaire (904) de l'élément tubulaire (910, 928) comprend une pluralité de sous-parties (904a, 904b), et chacune de la pluralité de sous-parties ayant un diamètre extérieur (936, 938, 992, 996) qui augmente de façon linéaire, selon un angle respectif (994, 998), dans une direction allant de la partie distale (906) vers la partie proximale (902).

**7.** Instrument d'ablation laparoscopique par micro-ondes selon la revendication 1, dans lequel la partie intermédiaire (904) de l'élément tubulaire (912, 924, 926) a un diamètre extérieur qui augmente de façon exponentielle dans une direction allant de la partie distale (906) vers la partie proximale (902).

**8.** Instrument d'ablation laparoscopique par micro-ondes selon la revendication 1, dans lequel la partie intermédiaire (904) de l'élément tubulaire (930) comprend une pluralité de sous-parties, et chacune de la pluralité de sous-parties ayant un diamètre extérieur (1002, 1004) qui augmente de façon exponentielle, à un taux d'augmentation respectif, dans une direction allant de la partie distale (906) vers la partie proximale (902).

**9.** Instrument d'ablation laparoscopique par micro-ondes selon la revendication 1, dans lequel la partie intermédiaire de l'élément tubulaire (914, 916) comprend une première sous-partie et une seconde sous-partie, la première sous-partie ayant un diamètre extérieur (946, 954) qui augmente de façon linéaire dans une direction de la partie distale (906) vers la partie proximale (902), et la seconde sous-partie ayant un diamètre extérieur (948, 952) qui augmente de façon exponentielle dans la direction de la partie distale (906) vers la partie proximale (902).

**10.** Instrument d'ablation laparoscopique par micro-ondes selon la revendication 9, dans lequel la première sous-partie et la seconde sous-partie sont agencées dans une direction allant de la partie distale (906) vers la partie proximale (902).

11. Instrument d'ablation laparoscopique par micro-ondes selon la revendication 10, dans lequel, à proximité d'une jonction entre la première sous-partie et la seconde sous-partie de l'élément tubulaire, le diamètre extérieur de la seconde sous-partie est supérieur au diamètre extérieur de la première sous-partie, formant ainsi un épaulement au niveau de la jonction entre la première sous-partie et la seconde sous-partie.

12. Instrument d'ablation laparoscopique par micro-ondes selon la revendication 8, dans lequel la première sous-partie et la seconde sous-partie de l'élément tubulaire sont agencées dans une direction allant de la partie proximale vers la partie distale ; le diamètre extérieur de la première sous-partie étant de préférence supérieur, à proximité d'une jonction entre la première sous-partie et la seconde sous-partie, au diamètre extérieur de la seconde sous-partie, formant ainsi un épaulement au niveau de la jonction entre la première sous-partie et la seconde sous-partie.

13. Instrument d'ablation laparoscopique par micro-ondes selon l'une quelconque des revendications précédentes, dans lequel la partie intermédiaire (904) de l'élément tubulaire (922) a un diamètre extérieur (974) qui augmente de façon linéaire dans une direction allant de la partie distale (906) vers la partie proximale (902), et dans lequel, à proximité d'une jonction entre la partie distale et la partie intermédiaire, le diamètre extérieur de la partie intermédiaire est supérieur à un diamètre extérieur de la partie distale, formant ainsi un épaulement (980) au niveau de la jonction entre la partie distale et la partie intermédiaire ; un diamètre extérieur de la partie proximale étant de préférence supérieur, à proximité d'une jonction entre la partie intermédiaire (904) et la partie proximale (902), au diamètre extérieur de la partie intermédiaire, formant ainsi un épaulement (978) au niveau de la jonction entre la partie intermédiaire et la partie proximale.

14. Instrument d'ablation laparoscopique par micro-ondes selon l'une quelconque des revendications précédentes, dans lequel la partie intermédiaire (904) de l'élément tubulaire (926) a un diamètre extérieur (986) qui augmente de façon exponentielle dans une direction allant de la partie distale (906) vers la partie proximale (902), et dans lequel, à proximité d'une jonction entre la partie distale et la partie intermédiaire, le diamètre extérieur de la partie intermédiaire est supérieur à un diamètre extérieur de la partie distale, formant ainsi un épaulement (988) au niveau de la jonction entre la partie distale et la partie intermédiaire.

15. Instrument d'ablation laparoscopique par micro-ondes selon la revendication 14, dans lequel, à proximité d'une jonction entre la partie intermédiaire (904) et la partie proximale (902) de l'élément tubulaire, un diamètre extérieur (970) de la partie proximale est supérieur au diamètre extérieur (986) de la partie intermédiaire, formant ainsi un épaulement (990) au niveau de la jonction entre la partie intermédiaire et la partie proximale.

To Microwave
Ablation
Generator

FIG. 1

FIG. 2

**FIG. 3**

FIG. 4

FIG. 5

**FIG. 6A**

**FIG. 6B**

EP 3 417 823 B1

**FIG. 7**

FIG. 8A

FIG. 8B

FIG. 9A

FIG. 9B

FIG. 9C

EP 3 417 823 B1

EP 3 417 823 B1

**FIG. 9D**

**FIG. 9E**

**FIG. 9F**

EP 3 417 823 B1

**FIG. 9J**

**FIG. 9K**

**FIG. 9L**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2545179 A

- US 20140290830 A1